# EUROPEAN PATENT SPECIFICATION

(11) **EP 4 028 549 B1**
(45) Date of publication and mention of the grant of the patent: **11.06.2025**
(21) Application number: 20767854.1
(22) Date of filing: 11.09.2020
(51) Int. Cl.: C12Q 1/6844, C12N 15/86

(54) **METHOD FOR THE PRODUCTION OF RAAV AND METHOD FOR THE IN VITRO GENERATION OF GENETICALLY ENGINEERED, LINEAR, SINGLE-STRANDED NUCLEIC ACID FRAGMENTS CONTAINING ITR SEQUENCES FLANKING A GENE OF INTEREST**
VERFAHREN ZUR HERSTELLUNG VON RAAV UND VERFAHREN ZUR IN-VITRO-ERZEUGUNG VON GENETISCH MANIPULIERTEN, LINEAREN, EINZELSTRÄNGIGEN ITR-SEQUENZ-HALTIGEN NUKLEINSÄUREFRAGMENTEN, DIE EIN GEN VON INTERESSE FLANKIEREN
PROCÉDÉ DE PRODUCTION DE RAAV ET PROCÉDÉ DE GÉNÉRATION IN VITRO DE FRAGMENTS D'ACIDE NUCLÉIQUE MONOBRIN, LINÉAIRES ET GÉNÉTIQUEMENT MODIFIÉS CONTENANT DES SÉQUENCES ITR FLANQUANT UN GÈNE D'INTÉRÊT

(30) Priority: 11.09.2019 EP 19196672
(43) Date of publication of application: 20.07.2022
(73) Proprietor: Universität Bielefeld, 33615 Bielefeld (DE)
(72) Inventor: MÜLLER, Kristian, 14469 Potsdam (DE); RADUKIC, Marco, 33334 Güterloh (DE)
(74) Representative: Grund, Martin
(86) International application number: PCT/EP2020/075486
(87) International publication number: WO 2021/048366

(56) References cited:
- EP-A1- 2 862 935
- WO-A1-2008/095927
- WO-A2-2008/016391
- DUCANI COSIMO ET AL: "Enzymatic production of 'monoclonal stoichiometric' single-stranded DNA oligonucleotides", NATURE METHODS (AUTHOR MANUSCRIPT), vol. 10, no. 7, 2 June 2013 (2013-06-02), New York, pages 647 - 652, XP055805469, ISSN: 1548-7091, Retrieved from the Internet <URL:http://www.nature.com/articles/nmeth.2503> DOI: 10.1038/nmeth.2503
- "Synthetic DNA, Chapter 7", 27 September 2016, HUMANA PRESS, article DUCANI COSIMO ET AL: "Enzymatic Synthesis of Single-Stranded Clonal Pure Oligonucleotides", pages: 93 - 103, XP093084406
- HAN PING ET AL: "Construction and Gene Expression Analysis of a Single-Stranded DNA Minivector Based on an Inverted Terminal Repeat of Adeno-Associated Virus", MOLECULAR BIOTECHNOLOGY, vol. 57, no. 4, 3 January 2015 (2015-01-03), Boston, pages 382 - 390, XP055645407, ISSN: 1073-6085, DOI: 10.1007/s12033-014-9832-3
- KINGA KARBOWNICZEK ET AL: "Doggybone(TM) DNA: an advanced platform for AAV production", CELL AND GENE THERAPY INSIGHTS, vol. 3, no. 9, 16 November 2017 (2017-11-16), pages 731 - 738, XP055669721, ISSN: 2059-7800, DOI: 10.18609/cgti.2017.074
- MARIA SCHN?DT ET AL: "DNA Minicircle Technology Improves Purity of Adeno-associated Viral Vector Preparations", MOLECULAR THERAPY-NUCLEIC ACIDS, vol. 5, 1 January 2016 (2016-01-01), pages e355, XP055406390, ISSN: 2162-2531, DOI: 10.1038/mtna.2016.60
- LINA LI ET AL: "Production and Characterization of Novel Recombinant Adeno-Associated Virus Replicative-Form Genomes: A Eukaryotic Source of DNA for Gene Transfer", PLOS ONE, vol. 8, no. 8, 1 August 2013 (2013-08-01), pages 1 - 14, XP055416248, DOI: 10.1371/journal.pone.0069879
- JOHNE R ET AL: "Rolling-circle amplification of viral DNA genomes using phi29 polymerase", TRENDS IN MICROBIOLOGY, ELSEVIER SCIENCE LTD., KIDLINGTON, GB, vol. 17, no. 5, 1 May 2009 (2009-05-01), pages 205 - 211, XP026095083, ISSN: 0966-842X, [retrieved on 20090415], DOI: 10.1016/J.TIM.2009.02.004
- YONGLUN LUO ET AL: "Efficient construction of rAAV-based gene targeting vectors by Golden Gate cloning", BIOTECHNIQUES, vol. 56, no. 5, 1 May 2014 (2014-05-01), US, XP055746425, ISSN: 0736-6205, DOI: 10.2144/000114169
- ALFRED PINGOUD ET AL: "Type II restriction endonucleases-a historical perspective and more", NUCLEIC ACIDS RESEARCH, vol. 42, no. 12, 30 May 2014 (2014-05-30), pages 7489 - 7527, XP055632911, ISSN: 0305-1048, DOI: 10.1093/nar/gku447

## Description

In a first aspect, the present invention relates to a method for the *in vitro* generation of genetically engineered, linear, single-stranded nucleic acid fragments containing two AAV inverted terminal repeat (ITR) sequences flanking a gene of interest (GOI), being essentially free of bacterial nucleic acid sequences. The method is based on a rolling circle amplification.

In a further aspect, the present invention provides an isolated, linear, single-stranded nucleic acid comprising viral nucleic acid fragments obtainable by a method according to present invention.

Moreover, the invention provides a plasmid comprising restriction enzyme recognition sequences flanking AAV ITR sequences flanking a gene of interest (GOI).

### Prior art

Adeno-associated virus (AAV), in particular, serotype 2, has gained tremendous popularity as a vector for gene therapy, but also as a platform for vaccine development and as a tool in pre-clinical research. That is, virus derived vectors represent one of the most popular gene delivery systems for mammalian cells. The interest in AAV vector particles is based on several beneficial features including apathogenicity, high stability, ability to transduce both dividing and non-dividing cells, long term gene expression in post-mitotic or slowly proliferating cells and low immunogenicity. In addition, recombinant AAV vectors lack an intrinsic integrase activity and are therefore defined as non-integrating vector systems. This is a main advantage compared to retro-/lentiviral vectors as it significantly reduces the risk of insertional mutagenesis. AAV vectors are typically produced from mammalian or insect cell cultures following sophisticated protocols.

Research and development of recombinant Adeno-associated virus (rAAV) vectors for clinical applications is coming more and more in focus raising interest in suitable techniques to produce rAAV preparations at high titer and improved purity.

The development is boosted by the recent approval of three AAV-based gene therapeutics for orphan diseases by regulatory bodies in the EU and the United States, namely Glybera, Luxturna and Zolgensma. AAV-based therapeutics for indications with higher prevalence, including hemophilia, are subject of ongoing clinical trials. In addition, basic research discloses more and more fields of application, for example, site directed gene therapy with rAAVs or tumor directed gene therapy.

One major drawback of rAAV therapeutics is the complex production method using biological agents in living cells required to mimic a lytic viral infection. Typically, immortalized, kidney-derived human cells are used to produce rAAV. Cultivation thereof is expensive and prone to failure. One possible risk arises from potential coinfection of the producer cells with other human or primate viruses. Depleting viral contaminants in the product is difficult, in particular, when the final therapeutic product is a virus like particle (VLP) or viral vector. Another problem is the presence of bacterial nucleic acid sequences packaged into AAV capsids (Chadeuf, G., et. Al., 2005, Molecular Therapy : The Journal of the American Society of Gene Therapy, 12(4), 744-753 and Lecomte, E., et al., 2015, Molecular Therapy - Nucleic Acids, 4(10), e260). These stem from the plasmids used for rAAV production in cell culture and are packaged during vector production.

They are likely derived from the plasmid encoding the recombinant virus sequences and a gene of interest or sequence of interest to be introduced into the capsid used for obtaining virus like particles for gene therapy applications. However, these bacterial sequences are potentially immunogenic. Therefore, to be able to comply with the increasing demand for rAAV and safety, the present methods for production of the same must be improved or new methods must be developed. This is particularly true for repeated therapeutic applications such as directed tumor gene therapy. In directed tumor gene therapy, a broad spectrum of tumor diseases shall be treated with high doses of rAAV, different to the presently approved gene therapeutic therapies directed to orphan diseases.

Wild-type AAV serve as a platform for the development of recombinant AAV also identified here as AAV vectors. AAV are a member of the family *Parvoviridae* and belong to the genus *dependoparvovirus.* AAV are composed of a single stranded DNA genome of about 4.7 kB packaged into a non-enveloped protein capsid, which is of icosahedral shape and consists of 60 monomers. The monomers are one of the viral capsid proteins VP1, VP2 or VP3. The about 4.5 kB single-stranded regulatory or coding DNA genome (plus inverted terminal repeats, ITRs) is bearing two gene cassettes (rep and cap) encoding non-structural (Rep proteins and assembly activating protein) and structural proteins (VP1, VP2, VP3). The AAV genome is flanked by ITRs, which serve as packaging and replication signals. AAV is unique in being dependent on a helper virus like adenovirus for progeny production.

In AAV vectors, viral open reading frames are replaced by foreign nucleic acids such as a transgene expression cassette or parts of the AAV nucleic acid sequence only. AAV vectors deliver a single stranded DNA encoding the sequence of interest or gene of interest flanked by the viral ITR structures. They are designed either in the natural genome conformation or as so-called self-complementary vector genomes. Vector genomes are packaged during vector production into natural occurring capsids (serotype/variants) or engineered capsids.

To produce rAAV for therapeutic applications in mammalian cell culture, the AAV genome is typically split into two plasmids, where one bears the two open reading frames of the AAV genome depleted of ITRs and the other plasmid carries the gene of interest flanked by ITRs, which mediate its encapsidation. A third plasmid provides further helper functions of the adenovirus (Matsushita, T., et al., 1998, Gene Therapy, 5(7), 938-945 and Xiao, X., Li, J., & Samulski, R. J., 1998, Journal of Virology, 72(3), 2224-2232.). The two non-ITR bearing plasmids can be combined into one helper plasmid. Several AAV serotypes are characterized and their varying tropism can be exploited in therapy for tissue specific gene delivery. Pseudotyping (pseudopackaging), a strategy to change the tropism of the AAV vectors, further broadens their therapeutic reach. On the genetic level, sequences for tissue-specific peptides can also be incorporated into the capsid for altered tropism.

The problem of packaging of bacterial sequences may be circumvented using the minicircle technology of plasmid factory, Bielefeld, Germany (Schnödt, M., et al., 2016, Molecular Therapy - Nucleic Acids, 5(8), e355) (*see also* EP 2 8620935). The system is based on a bacterial plasmid including the GOI to be packaged into the capsids, flanked by ITRs and recombinase recognition sequences. During minicircle production, the parental plasmid is modified by a recombinase to a double stranded, covalently closed "minicircle" containing just the ITRs, a recombinase scar and the gene of interest. The minicircle then replaces the GOI plasmid during AAV vector production. Generation of minicircles from the helper plasmids is also possible. The described minicircle technology should overcome the problem of packaging bacterial sequences remaining in the DNA to be included into the capsids.

Another technique has been described for obtaining rAAVs essentially free of bacterial sequences. Scott, et.al., 2015 Human Vaccines & Immunotherapeutics, 11(8), 1972-1982 describe novel synthetic circular DNA vaccines, termed Doggybone DNA. This system is based on applying a rolling circle amplification of a DNA eventually obtaining double stranded DNA, which is used for the rolling circle amplification again, thereby providing exponential amplification. In a final step, a protelomerase resolves the amplification product to double-stranded fragments with covalently closed telomeric ends. The company Touchlight further develops and markets this technology. Therein, the use of two enzymes, namely the phi 29 DNA polymerase and a protelomerase to generate covalently closed, linear DNA constructs named Doggybone DNA or dbDNA is disclosed. Recently, the production of AAV particles using this technology has been described, see Karbowniczek, K., et. Al., 2017, Cell and Gene Therapy Insights, 3(9), 731-738.

This method is based on the covalently closed DNA construct being present as a circular DNA construct. Pending patent applications with respect to the described Doggybone system include WO 2018/033730 A1, WO 2016/122129 A1, WO 2016/034849 A1, WO 2012/017210 A1 and WO 2010/086626 A1.

Both techniques to produce rAAV essentially free of bacterial sequences known in the prior art-namely minicircle and Doggybone technology-provide synthetic DNA molecules different to the DNA molecules needed for rAAV genome amplification and packaging into the viral capsids, which are linear, single stranded molecules. Thus, production does not follow the natural route and in both cases might be subop-timal. Moreover, minicircle technology relies on DNA amplification in bacteria, where GC-rich repeat sequences, like ITRs, are known to be unstable. Further, Doggybone technology relies on exponential DNA amplification, possibly leading to the accumulation of amplification errors in the product, especially arising from GC-rich repeat sequences like ITRs.

Inefficient and expensive rAAV production results in the desire to provide a single stranded DNA, which can be an AAV genome or a gene of interest (GOI) or sequences of interest (SOI), for improved rAAV production.

Previous approaches relating to dsAAV and ssAAV vector constructs are described in WO 2008/016391 as well as Ping et al., 2015.

Rolling circle amplification is described in the art. Namely rolling circle amplification refers to a process of unidirectional nucleic acid amplification from a circular DNA or RNA template that can synthesize multiple concatenated copies, or more pecise reverse complement copies, of the template sequence, such as plasmids, genomes of bacteriophages, and the circular RNA genome of viroids. Typically, isothermal DNA amplification techniques are used for rolling circle amplification in which a polymerase continuously adds single nucleotides to a primer hybridized to the template resulting in a single-stranded nucleic acid with tens to hundreds of copies of the circular template. Useful polymerases applicable in rolling circle amplification are DNA polymerase obtainable from bacteriophages such as the phi 29 DNA polymerase or the BST and the vent (exo-) DNA polymerase for DNA amplification as well as the T7 RNA polymerase for RNA amplification.

Often the rolling circle amplification technique is applied for determining and detecting targets with low abundance in a sample.

From the above, it is clear that there is a continuous demand for the production of nucleic acid fragments for the introduction into virus like particles or empty capsids in particular of AAV. Further, the provision of a method for the efficient production of rAAV is desired.

### Brief description of the present invention

In a first aspect, the present invention relates to a method for the *in vitro* generation of genetically engineered, linear, single-stranded nucleic acid (ssNA) fragments containing two adeno-associated viral inverted terminal repeat (AAV ITR) sequences flanking a gene of interest (GOI), being essentially free of bacterial nucleic acid sequences, comprising the steps of:
a) Providing a bacterial nucleic acid plasmid containing a nucleic acid fragment containing the two AAV ITR sequences and the GOI;
b) Enzymatic digestion of the plasmid with a first restriction enzyme (1) or a combination of at least two restriction enzymes (1) and (4) generating the double stranded nucleic acid fragment with terminal compatible ends;
c) Isolation of the linear double stranded nucleic acid fragment containing the nucleic acid fragment with AAV ITR sequences and GOI;
d) Recirculation of the isolated linear double stranded nucleic acid fragment of step c) to obtain a double stranded circular nucleic acid fragment joint at the compatible ends formed in step b);
e) Amplification of the circulated double stranded fragment of d) containing two AAV ITRs and the GOI by a nucleic acid amplification technique generating single-stranded nucleic acids comprising at least two covalently linked copies (linked reverse complement copies) of the template strand;
f) Enzymatic digestion of the amplification product obtained in step e) with a second restriction enzyme (2) or at least two different restriction enzymes (2) and (3) to obtain linear ssNA sequences comprising said nucleic acid fragment of the two AAVITR sequences and the GOI; and
g) Purification of said linear ssNA fragment obtained in step f) comprising the nucleic acid fragment of the two AAV ITR sequences and the GOI.

In a further aspect, an isolated linear ssNA fragment obtainable by a method according to the present invention is provided.

Finally, a plasmid comprising the following nucleic acid sequence elements:
a) A recognition sequence for a first restriction enzyme (1) generating ends, like sticky ends, being compatible to the generated ends of g);
b) Downstream a recognition sequence for a second restriction enzyme (2) and said recognition sequence being different to the recognition sequence of a) and g), whereby the recognition sequence is selected from the sequences recognized by Type IIS enzymes which would generate a 3'-OH towards the functional ITR directly upstream or inside of the ITR sequence of c);
c) Downstream a functional AAV ITR sequence;
d) Downstream the GOI;
e) Downstream a functional AAV ITR sequence being identical or different to the ITR sequence of c);
f) Downstream a recognition sequence for a third restriction enzyme (3) different to the recognition sequence of a) and g);and the recognition sequence is the reverse complement of the sequence of b);
Downstream a recognition sequence for a fourth restriction enzyme (4) generating ends, being compatible to the ends generated in a); whereby said fourth restriction enzyme (4) may be identical or different to the first enzyme (1). The plasmid, the rAAV and the other ssNAs described herein are particularly suitable in gene therapeutic methods, in particular, AAV based gene therapy. Treatment eligible diseases include monogenic disorders like Sickle cell anemia, Severe Combined Immunodeficiency (ADA-SCID / X-SCID), Cystic fibrosis, Hemophilia, Duchenne muscular dystrophy, Huntington's disease, Parkinson's, Hypercholesterolemia, Alpha-1 antitrypsin, Chronic granulomatous disease, Fanconi Anemia and Gaucher Disease, as well as polygenic disorders like Heart disease, Cancer, Diabetes type I and type II, Schizophrenia and Alzheimer's disease. Further possible treatments include communicable diseases, infections, virus diseases, neoplasms, immune system diseases, syndromes, digestive system diseases, gastrointestinal diseases, respiratory tract diseases, immunologic deficiency syndromes, sexually transmitted diseases, skin diseases, lung diseases, liver diseases, hematologic diseases, metabolic diseases, immunoproliferative disorders, leukemia, lymphatic diseases, endocrine system diseases, mental disorders, psychotic disorders, genital diseases, intestinal diseases, brain diseases, musculoskeletal diseases, colonic diseases, urologic diseases, neurologic manifestations, rectal diseases, congenital abnormalities, blood coagulation disorders, hemostatic disorders, hemorrhagic disorders, bone marrow diseases, prostatic diseases and neuromuscular diseases.

### Brief description of the drawings

Figure 1: Fig.1 is a scheme showing the different steps of a method according to the present invention.
Figure 2: Fig.2 shows the production and biological activity of the fragments according to the present invention (Fig. 2a and 2b) and of virus particles obtained according to the present invention.

### Detailed description

The invention is defined by the appended claims.

The present inventors aim to provide a new method for the *in vitro* generation of genetically engineered, linear, single-stranded nucleic acid (ssNA) fragments which are useful for incorporation into viral vectors. The method according to the present invention is based on a rolling circle amplification resulting in ssNA fragments. That is, the present invention relates to a method for the *in vitro* generation of genetically engineered, linear, ssNA fragments containing two AAV inverted terminal repeat (ITR) sequences flanking a gene of interest (GOI), being essentially free of bacterial nucleic acid sequences, comprising the steps of:
a) Providing a bacterial nucleic acid plasmid containing a nucleic acid fragment containing the two AAV ITR sequences and the GOI;
b) Enzymatic digestion of the plasmid with a first restriction enzyme (1) or a combination of at least two restriction enzymes (1) and (4) generating the double stranded nucleic acid fragment with terminal compatible ends;
c) Isolation of the linear double stranded nucleic acid fragment containing the nucleic acid fragment with AAV ITR sequences and GOI;
d) Recirculation of the isolated linear double stranded nucleic acid fragment of step c) to obtain a double stranded circular nucleic acid fragment joint at the compatible ends formed in step b);
e) Amplification of the circulated double stranded fragment of d) containing two AAV ITRs and the GOI by a nucleic acid amplification technique generating single-stranded nucleic acids comprising at least two covalently linked copies (linked reverse compliment copies) of the template strand;
f) Enzymatic digestion of the amplification product obtained in step e) with a second restriction enzyme (2) or two different restriction enzymes (2) and (3) to obtain linear ssNA sequences comprising said nucleic acid fragment of the two AAV ITR sequences and the GOI; and
g) Purification of said linear ssNA fragment obtained in step f) comprising the nucleic acid fragment of the two AAV ITR sequences and the GOI.

Disclosed is also a method for the *in vitro* generation of genetically engineered, linear, ssNA fragments containing two viral inverted terminal repeat (ITR) sequences flanking a gene of interest (GOI), being essentially free of bacterial nucleic acid sequences, comprising the steps of:
a) Providing a bacterial nucleic acid plasmid containing the nucleic acid fragment containing the two viral ITR sequences and the GOI,
b) Enzymatic digestion of the plasmid with a first restriction enzyme (1) or a combination of at least two restriction enzymes (1) and (4) generating compatible ends with the double stranded nucleic acid fragment,
c) Isolation of the linear double stranded nucleic acid fragments containing the nucleic acid fragment with viral ITR sequences and GOI;
d) Recirculation of the isolated linear double stranded nucleic acid fragments of step c) to obtain double stranded circular nucleic acid fragments joint at the compatible ends formed in step b);
e) Amplification of single stranded nucleic acid fragments comprising more than one nucleic acid fragments with the two viral ITR sequences and the GOI from the double stranded circular nucleic acid fragment of d) as the template whereby more than one nucleic acid fragments being covalently linked with each other;
f) Enzymatic digestion of the amplification product obtained in step e) with a second restriction enzyme (2) or at least two different restriction enzymes (2) and (3) generating compatible ends to obtain linear ssNA sequences comprising said nucleic acid fragment of the two viral ITR sequences and the GOI; and
g) Purification of said linear ssNA fragment obtained in step f) comprising the nucleic acid fragment of the two viral ITR sequences and the GOI.

That is, the present inventors recognized that applying the rolling circle amplification to a specifically designed bacterial plasmid with the specific functions as described allows to obtain linear, ssNA fragments containing functional viral ITR sequences and a gene of interest essentially free, in particular, free of any bacterial sequences increasing safety and apathogenicity of resulting viral particles. The linear ssNA fragments retain their biological function and are suitable for the production of recombinant virus particles. In particular, *in vitro* production of viral particles from virus like particles and production of rAAV *in vitro* with suitable load of nucleic acid fragments is possible. Said load include the linear ssNA fragment according to the present invention. Namely, the fragment according to the present invention obtainable by the method according to the present invention represents an optimal substrate for *in vitro* packaging, thus, obtaining recombinant virus particles, like rAAV. Using the linear ssNA fragments according to the present invention allows to reduce the production process and the costs of the production as well as increasing safety aspects etc.

As used herein, the term "genetically engineered" refers to an artificially modified nucleic acid molecule obtained by recombinant nucleic acid technology.

The term "nucleic acid" includes DNA, RNA as well as variants thereof such as siRNA, miRNA as well as other known nucleic acid variants like PNA, lock nucleic acid (LNA) as well as glycol nucleic acid (GNA) and triose nucleic acid (TNA) where appropriate.

The term "ITR sequence(s)" refers to the nucleic acid sequence serving as the viral origin of replication being also required for packaging. That is, a functional ITR includes the packaging and replication signals. Typically, the ITR are present at the ends of the viral genome, like in the Adeno-associated virus genomes.

According to the present invention, when identifying the ITR sequences, the ITR sequences are based on viral sequences as described in the art or fragments thereof, whereby said fragments according to the present invention are ITR fragments having the same major functionality as the complete ITR sequences, namely, allowing packaging and replication. For example, minimal ITR sequences are described by Zhou, Q., et. al., 2017, Scientific Reports, 7(1), 5432).

The term "substantially free of bacterial nucleic acid sequences", as used herein, identifies that the nucleic acid fragments according to the present invention do not contain any bacterial nucleic acid sequences, namely, nucleic acid sequences stemming from the bacterial plasmid or contain only very few bacterial nucleic acid bases, like at most 40, 30, 25, 20, 15, 12, 10, 9, 8, 7, 6, 5, 4, 3, 2 or 1 nucleic acid bases.

In particular, the full absence of bacterial sequences increases safety in the production of rAAV by *in vitro* packaging, in particular, these recombinant virus particles, like rAAV, are useful in gene therapy.

As used herein, the terms "compatible ends" and "sticky ends" refer to five prime and three prime ends of double stranded nucleic acid fragments wherein at least one nucleic acid overhangs the double stranded fragment, namely, being an unpaired nucleic acid of a single strand, thus, representing sticky ends. These compatible ends are typically generated by restriction enzymes. With a complementary base pair or complementary base pairs, these overhangs or sticky ends represent the compatible ends, capable of forming double stranded nucleic acids. Further, the compatible ends also include blunt ends, namely, double stranded fragments wherein both strands terminate in a base pair.

The term" "restriction enzyme" includes suitable enzymes digesting double stranded nucleic acid molecules, which can be conventional restriction enzymes known in the art or designer restriction enzymes which can be based on CRISPR enzymes, TALENs or zinc finger nucleases and homing endonucleases.

The terms "Type IIS" or "Type IIS enzymes" refer to restriction endonucleases that recognize a specific DNA sequence and cleave shifted, outside of the recognition sequence, regardless of their target symmetry, enzyme structure complexity or cofactor requirements.

The term "amplification" refers to the repeated duplication or templated synthesis of nucleic acids. Amplification is possible by various methods including polymerase chain reaction, rolling circle amplification and (loop mediated) isothermal amplification.

The terms "complementary sequence" or "complementary" refer to a nucleic acid molecule that can pair with another given nucleic acid molecule to form a double-stranded structure by matching base pairs.

The terms "vector particle", or "vector" or "virus particle" identify a viral capsid containing viral, transcriptionally active nucleic acid, like DNA.

The terms "particle" or "virus like particle (VLP)" identify a capsid without nucleic acid, like DNA, or containing non-viral nucleic acid, like DNA.

The term "AAV" refers to the Adeno-associated virus or derivatives thereof including recombinant AAV vector particles wherein the term "AAV wildtype particle" designates the Adeno-associated virus capsid as it occurs in nature without DNA or containing DNA. The term "recombinant AAV" or "recombinant vector particle" is an AAV wherein the genome of the virus is substituted with a vector genome, namely, a foreign DNA to be introduced into a cell. Said recombinant AAV is also referred to as "rAAV". The term "AAV" or the respective recombinant AAV vector particle and AAV wildtype particle include at least 13 different serotypes known in the art. The AAV of human serotype 2 is also mentioned as AAV 2 or AAV 2 particle or AAV 2 vector particle. AAV 2 is a preferred embodiment. The term AAV and its variations also refer to pseudo-typed, chimeric or rationally modified capsids.

The term *"in vitro"* as used herein refer to a cell-free method of obtaining the ssNA according to the present invention. No incubation with, or transformation of living biological material etc. is required starting from the plasmid to obtain the ssNA according to the present invention.

As used herein, the phrase "single-strand purity" refers to the fraction of sequences in the sample that are single-stranded as opposed to sequences that are double-stranded by hybridization with a counter-strand.

In an embodiment of the present invention, the genetically engineered, linear, ssNA fragment according to the present invention containing the two viral inverted terminally repeat sequences flanking a gene of interest is free of bacterial nucleic acid sequences. Thus, it is possible to increase safety of the virus particles containing the same as well as increase the feasibility for drug approval.

The vector genome, like the foreign DNA to be introduced into cells, present in the recombinant vector particle or recombinant AAV, may be the natural virus genome, like the natural AAV genome or, in another embodiment, a foreign DNA containing a GOI or SOI to be introduced into cell.

Typical examples of a gene of interest or a sequence of interest to be introduced into the cell, for example in gene therapy, include genes coding for: adhesion molecules, antigens, antisense DNA, cell cycle arrestor, drug resistance marker, cytokines, enzyme replacements, growth factors, hormones, markers, oncogene regulators, porins, ion channels, transporters, receptors, replication inhibitors, ribozymes, siRNA, suicide genes, transcription factors, tumor suppressors, antibody derived sequences and antibody mimetics such as DARPins, Affibodies, Anticalins, Adenectins, Affilins, and Nanobodies.

In an embodiment of the present invention, the method for the *in vitro* generation of linear single stranded viral nucleic acid fragments is a method using a restriction enzyme (1) and/or (4) generating sticky ends. These restriction enzymes (1) and/or (4) include type II enzymes, like Pstl, type IIS endonucleases and designer endonucleases.

In another embodiment of the method according to the present invention, the restriction enzyme (2) and/or (3) being different to the restriction enzymes (1) and (4) is a blunt-end cutting restriction enzyme, e.g. of type II, like Pvull, or type IIS or a designer endonuclease. Alternatively, the second restriction enzyme may be an enzyme generating sticky ends.

In another embodiment, the sequence for the restriction enzyme (2) and (3) and the respective adjacent sequences are planned such that they can form a double stranded DNA when just one DNA strand is present and that this double stranded site serves as a recognition site for a restriction enzyme and optionally remains double stranded up to the nearby placed ITR sequences.

In another embodiment, the restriction enzyme recognizing the formed double stranded part within the single-strand DNA adjacent to the ITRs is a Type IIS enzyme which cuts the single-stranded DNA folded to a double strand up to the ITR such that a 3'-OH is generated directly at the end or inside of one ITR. The number and composition of nucleotides between the DNA folded to a recognition sequence and the ITR sequence can be adjusted. This method has the advantage that ITR-GOI fragments can be generated by digestion of the ssDNA on the one hand independent of the ITR sequences and on the other hand with different ITR lengths up to the full ITR length.

In an embodiment, restriction enzymes (1) and (4) may be used to digest the double-stranded nucleic acid into fragments with compatible ends. The same is possible for the restriction enzymes (2) and (3).

In an embodiment of the present invention, the method for the *in vitro* generation of linear single-stranded genetically-engineered viral nucleic acid fragments is a method wherein the plasmid containing viral nucleic acid fragments containing the sequence elements:
a) A recognition sequence for a first restriction enzyme (1) generating ends, like sticky ends, being compatible to the generated ends of g);
b) Downstream a recognition sequence for a second restriction enzyme (2) being able to complement with the sequence in f) and said recognition sequence being different to the recognition sequence of a) and g);
c) Downstream a functional AAV **ITR** sequence;
d) Downstream the GOI;
e) Downstream a functional AAV ITR sequence being identical or different to the ITR sequence of c);
f) Downstream a recognition sequence for a third restriction enzyme (3) being able to complement with the sequence in b) but different to the recognition sequence of a) and g); whereby the third restriction enzyme (3) may be identical or different to the second restrictions enzyme (2) of b);
g) Downstream a recognition sequence for a fourth restriction enzyme (4) generating ends, being compatible to the ends generated in a); whereby said fourth restriction enzyme (4) may be identical or different to the first enzyme (1); whereby, if restriction enzymes are used, that can cut at different distances relative to the recognition site, the recognition sequences in a) and b) as well as in f) and g) can be at the same position and up to all recognition sequences can be identical.

That is, the method for the *in vitro* generation of linear single-stranded genetically-engineered viral nucleic acid fragments is a method wherein the plasmid containing viral nucleic acid fragments containing the sequence elements:
a) A recognition sequence for a restriction enzyme (1) generating ends, like sticky ends, being compatible to the generated ends of g);
b) Downstream a recognition sequence for a restriction enzyme (2) generating ends, like sticky ends, being compatible to the generated ends of f), and said recognition sequence being different to the recognition sequence of a) and g);
c) Downstream a functional AAV ITR sequence;
d) Downstream the GOI;
e) Downstream a functional AAV ITR sequence being identical or different to the functional ITR sequence of c);
f) Downstream a recognition sequence for a restriction enzyme (3) generating ends being compatible generated ends of b) but different to the ends of a) and g), whereby the restriction enzyme (3) may be identical or different to the restriction enzyme (2) of b);
g) Downstream a recognition sequence for a restriction enzyme (4) generating ends, being compatible to the ends generated in a.) whereby said restriction enzyme (4) may be identical or different to the restriction enzyme (1); whereby if restriction enzymes are used, that can cut at different distances relative to the recognition site, the recognition sequences in a) and b) as well as in f) and g) can be at the same position and up to all recognition sequences can be identical.

In another embodiment, the recirculation step of the nucleic acid fragment in step b) may be conducted by a ligase, in particular, a T4 ligase. The skilled person is well aware of suitable enzymes and conditions for recirculation of the double-stranded nucleic acid fragment at the compatible ends obtained after digestion with the restriction enzymes.

In another embodiment, the method includes the step of providing an oligonucleotide, being a DNA or RNA oligonucleotide with free or modified 5' ends, to the recirculated isolated nucleic acid obtained in step d) and hybridizing said oligonucleotide specifically to a single nucleic acid strand of said nucleic acid. Alternatively, the nucleic acid obtained in step d) contains a nicking site allowing for nicking specifically of one strand of said nucleic acid. The skilled person is well aware of suitable nicking enzymes, like nicking endonuclease, e. g. Nt.BspQI.

In another embodiment, the nucleic acid obtained in step d) is nicked on one strand by a suitable nicking enzyme and then subjected to an exonuclease like T5 exonuclease, leaving behind a circular single-stranded template to which an oligonucleotide being an DNA or RNA oligonucleotide with free or modified 5' ends, is provided.

Another embodiment of the present invention relates to a method wherein hybridization of the oligonucleotide is conducted by heat denaturation of the nucleic acid obtained in step d) and subsequent cooling of the reaction mixture.

In another embodiment, the method including the steps of i) isolation of the double-stranded nucleic acid obtained in step d) and/or ii) purification of the linear single-stranded nucleic acid in step h), is conducted by electrophoreses or ion exchange chromatography or silica adsorption chromatography.

In another embodiment, the present invention relates to a method wherein the amplification step e) is performed enzymatically by a polymerase. The polymerase is in a preferred embodiment the phi29 polymerase. The amplification step may be an isothermal reaction, which avoids thermal cycling.

That is, a preferred embodiment relates to a method wherein the amplification step e) is performed in the presence of the phi29 polymerase as isothermal nucleic acid amplification, in particular, isothermal DNA amplification.

In another embodiment, a single-strand binding protein (SSB) is present during amplification. The skilled person is well aware of suitable SSB, like T4 Gene 32 Protein.

Further, the present invention relates to a method wherein in step e) the product obtained by amplification of the single stranded circular DNA fragment is heat denaturated and cooled to facilitate hybridization of adjacent inverted terminal repeat and restriction site sequences to form a double-stranded hairpin containing a functional restriction site. This double-stranded hairpin containing the functional restriction site is particularly useful for further processing.

In a further embodiment, hybridization of the product obtained by amplification of the single stranded circular DNA fragment results in formation of a self-hydrolyzing deoxyribozyme useful for further processing.

In a further embodiment, an isolated linear ssNA comprising viral nucleic acid fragments obtainable by a method according to the present invention is provided. Said isolated linear single-stranded nucleic acid, in particular, a DNA genome, is useful for the preparation of recombinant viruses, in particular, rAAV, for medical applications, in particular, gene therapy.

The isolated linear ssNA has a single-strand purity of at least 80%, like at least 85%, 88%, 90% or more. The purity may be above 92% like 95%.

Further, the present disclosure provides a method for the production of recombinant virus particles, in particular, recombinant AAV particle (rAAV) comprising the step of packaging of the linear ssNA comprising viral nucleic acid fragments according to claim 10, or the step of packaging of the linear ssNA fragment comprising viral nucleic acid fragments, like DNA fragments, obtainable by a method according to the present invention into virus like particle, i) by transfecting said ssNA in cells containing the additional genes and molecules for obtaining the recombinant virus particle, in particular, rAAV, ii) by transfecting said ssNA and additional plasmids containing helper functions or iii) by transfecting said ssNA and co-infecting with viruses providing helper functions. Further, recombinant virus particles, like rAAV, can be produced cell-free, i.e. without any eukaryotic cells, by introducing the ssNA according to the present invention into VLP, like AAV capsids, produced in prokaryotic cells or by chemical synthesis.

The method for the production of recombinant virus particles, in particular, rAAV, can be performed by known means. For example, the isolated genetically engineered linear ssNA fragment according to the present invention or obtainable by a method according to the present invention is included into a virus-like particle. It is particularly preferred that the method is an *in vitro* method for obtaining rAAV useful in gene therapy. *In vitro* generation and *in vitro* packaging increase the safety of the product obtained. For example, viral capsids not containing any DNA may be produced in suitable cells and the nucleic acid fragment according to the present invention is introduced into said empty viral capsids at a later time point. Suitable cells to produce empty capsids include human kidney derived cells, other human cells, hamster ovary cells, other mammalian cells, yeast, insect cells, archaea and bacterial cells, algae cells and other plant cells. In an embodiment of the method according to the present invention for the production of recombinant virus particle, said method is a cell free production of viruses, in particular, AAV. In another aspect, the recombinant AAV itself is disclosed obtainable by a method according to the present invention for producing recombinant virus particle. Said rAAV contain the isolated genetically engineered linear ssNA according to the present invention.

Further, the present invention provides a plasmid comprising the following nucleic acid sequence elements:
a) A recognition sequence for a first restriction enzyme (1) generating ends, like sticky ends, being compatible to the generated ends of g);
b) Downstream a recognition sequence for a second restriction enzyme (2) and said recognition sequence being different to the recognition sequence of a) and g), whereby the recognition sequence is selected from the sequences recognized by Type IIS enzymes which would generate a 3'-OH towards the functional ITR directly upstream or inside of the ITR sequence of c);
c) Downstream a functional AAV ITR sequence;
d) Downstream the GOI;
e) Downstream a functional AAV ITR sequence being identical or different to the ITR sequence of c);
f) Downstream a recognition sequence for a third restriction enzyme (3) different to the recognition sequence of a) and g), and the recognition sequence is the reverse complement of the sequence of b);
g) Downstream a recognition sequence for a fourth restriction enzyme (4) generating ends, being compatible to the ends generated in a); whereby said fourth restriction enzyme (4) may be identical or different to the first enzyme (1).

That is, the present disclosure further provides a plasmid comprising the following nucleic acid sequence elements:
a) A recognition sequence for a first restriction enzyme (1) generating ends, like sticky ends, being compatible to the generated ends of g);
b) Downstream a recognition sequence for a second restriction enzyme (2) generating ends, like sticky ends, being compatible to the generated end of f) and said recognition sequence being different to the recognition sequence of a) and g);
c) Downstream a functional ITR sequence;
d) Downstream the GOI;
e) Downstream a functional ITR sequence being identical or different to the functional ITR sequence of c);
f) Downstream a recognition sequence for a third restriction enzyme (3) generating ends generating ends being compatible to the generated ends in b) but different to the recognition sequence of a) and g); whereby the third restriction enzyme (3) may be identical or different to the second restrictions enzyme (2) of b);
g) Downstream a recognition sequence for a fourth restriction enzyme (4) generating ends, being compatible to the generated in a) whereby said fourth restriction enzyme (4) may be identical or different to the first enzyme (1) whereby if restriction enzymes are used, that can cut at different distances relative to the recognition site, the recognition sequences in a) and b) as well as f) and g) can be at the same position and up to all recognition sequences can be identical .

In an embodiment, the plasmid comprises the following nucleic acid sequence elements:
a) A recognition sequence for a first restriction enzyme (1) generating ends, like sticky ends, being compatible to the generated ends of g);
b) Downstream a recognition sequence for a second restriction enzyme (2) and said recognition sequence being different to the recognition sequence of a) and g) whereby the recognition sequence is selected from the sequences recognized by Type IIS enzymes which would generate a 3'-OH towards the functional ITR directly upstream or inside of the ITR sequence of c);
c) Downstream a functional ITR sequence;
d) Downstream the GOI;
e) Downstream a functional ITR sequence being identical or different to the functional ITR sequence of c);
f) Downstream a recognition sequence for a third restriction enzyme (3) different to the recognition sequences of a) and g); and the recognition sequence is the reverse complement of the sequence of b);
g) Downstream a recognition sequence for a fourth restriction enzyme (4) generating ends, being compatible to the ends generated in a) whereby said fourth restriction enzyme (4) may be identical or different to the first enzyme (1).

The plasmid according to the present invention is for example a plasmid wherein the recognition sequence of the restriction enzyme (2) and (3) is selected from the sequence set of Type IIS enzymes selected from Aarl, Acc36I, AcIWI, Acul, Alw261, AlwI, AsuHPI, BbsI, BbvI, BccI, BceAI, BciVI, BcoDI, BfuAI, Bful, Bmrl, BmsI, Bmul, Bpil, Bpml, BpuEI, Bsal, Bse3DI, BseGI, BseMII, BseRI, BseXI, BsgI, BsIFI, BsmBI, BsmFI, Bso31I, BspCNI, BspMI, BspPI, BspQI, BspTNI, BsrDI, Bst6l, BstF5I, BstMAI, BstV11, BstV2I, Bsul, BtgZI, BtsCI, BtsIMutI, BtsαI, Bvel, Csel, Eam1104I, Earl, Ecil, Eco57I, Esp3l, FaqI, Faul, Fokl, Gsul, Hgal, Hphl, HpyAV, Lgul, Lsp1109I, Lwel, Mboll, Mfel, Mlyl, Mmel, NmeAIII, PciSI, Plel, PpsI, Sapl, Schl, SfaNI, TaqII, TspDTI or TspGWI.

The plasmid may contain additionally a sequence for a nicking enzyme, like a nicking endonuclease. Said recognition sequence may be preferably present in the GOI sequence. The same is true for the plasmid described in the method according to the present invention.

The plasmid may contain additionally a sequence for a self-hydrolyzing deoxyribozyme. Said deoxyribozyme sequence may be preferably present downstream of the first recognition sequence for restriction enzyme (1) and may also be present upstream of the recognition sequence for a fourth restriction enzyme (4) where its sequence may or may not be inverted compared to its first occurrence. When the sequence or sequences for a self-hydrolyzing deoxyribozyme are present, restriction enzyme recognition sequences for a second (2) and third (3) restriction enzyme may be omitted. Sequences for self-hydrolyzing deoxyribozyme present on the plasmid can be identical or dissimilar. The same is true for the plasmid described in the method according to the present invention.

In a further embodiment the plasmid contains the recognition sequence of the restriction enzyme (2) and (3) being located to cut directly upstream or downstream, respectively, of the first or last respective nucleotide of the ITR sequences.

The sequence composition of the plasmid according to the present invention allows to generate a linear ssNA fragment containing no or being essentially free of bacterial nucleic acid sequences. In particular, the plasmid according to the present invention is particularly suitable for the *in vitro* generation of said ssNA containing two viral ITR sequences flanking a GOI according to the present invention.

In a further embodiment, the sequence elements provided by the structure of the plasmid according to the present invention free of bacterial nucleic acid can be prepared by solid-phase chemical synthesis, resulting in a linear ssDNA. Further, the linear ssDNA can be circularized by a suitable ssDNA ligase like CircLigase (Lucigen), serving as a template for rolling circle amplification according to the present invention.

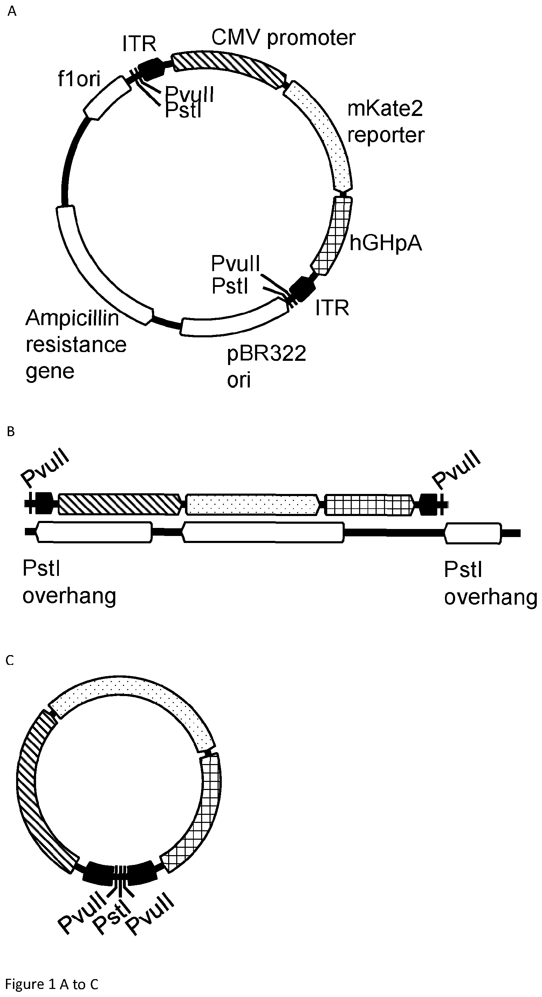

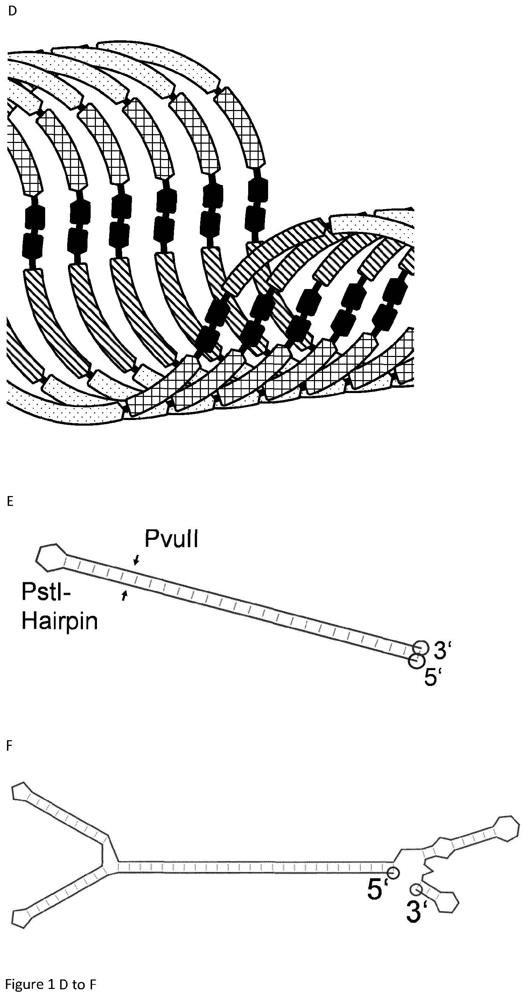

The invention will be described further referring to figure 1.

Figure 1 provides an overview of the vector plasmid and the various steps of the method for the *in vitro* generation of genetically engineered, linear, ssNA fragments containing two viral inverted terminal repeat (ITR) sequences flanking a gene of interest (GOI) according to the present invention. Scheme (A) depicts the vector plasmid useful for synthetic genome production. It contains wild type ITRs of AAV 2 shown in black. This type of ITR is present in most of AAV vector plasmids presently used. The two ITR sequences are flanking the GOI in the present case a promotor (hatched), followed by the coding sequence of a fluorescence reporter protein (dotted), further followed by a polyadenylation signal sequence (gridded). Recognition sites for restriction enzymes Pstl and Pvull are given as an ITR prefix or suffix for excision and recirculation as well as rolling circle product resolution.

In the present case, the ITR prefix contains a Pstl site, thus, allowing enzymatic digestion according to step b) of the method according to the present invention. Scheme (B) shows the result of the Pstl digestion, which generates two fragments, of which only the fragment containing the GOI is processed further.

After isolation of the fragment containing the GOI, recirculation is achieved with the sticky ends generated by Pstl, thus, obtaining a circular double stranded template for rolling circle amplification as shown in scheme (C) of figure 1. That is, digestion with the first restriction enzyme (1) and forth restriction enzyme (4), here both Pstl, yields a backbone fragment composed of bacterial sequences which are discarded and the GOI fragment. The GOI fragment is recirculated in step d) of the method according to the present invention by self-ligation to yield a double-stranded nucleic acid fragment shown in scheme (C). This template contains two adjacent ITR's in a head to head reverse complementary configuration. One Pstl site and the two Pvull sites are preserved. The two Pvull sites correspond to the restriction sites for the second restriction enzyme (2) and third restriction enzyme (3).

Rolling circle amplification of step e) of the circular template depicted in scheme (C) with a single primer produces single stranded high molecular weight multimers of the AAV synthetic genome with a single polarity shown in (D). The multimers are present as covalently bonded monomers of the ssNA containing the two viral ITR sequences flanking the GOI.

The head to head nature of the amplicon leads probably to hairpin formation at the recirculation scar in which the purposely planned sequence of the adjacent strands complement each other to generate a double stranded restriction site, here Pvull, that is used to release the genome monomer with the respective restriction enzyme. Scheme (E) illustrates a cutout of this hairpin structure shown together with the Pstl hairpin and the Pvull restriction site. Even if the two ITR's form a Y-shaped structure as shown in scheme (F), a short restriction enzyme recognition side hairpin probably forms between two ITR's and suffices for cleavage. It is noted that the secondary structure of the DNA obtained after the rolling circle amplification suitable for cutting by the restrictions enzyme as described is distinct from the Y-shape structure proposed for ITR's *in vivo.* The enzymatic digestion of the amplification product results in single stranded linear nucleic acid fragments. Notably, the reverse complementary ITR sequences may stay double stranded after excision of the monomer until further processing.

Thus, the method according to the present invention is different to the methods described so far. The linear ssNA fragment obtained after the second digestion is purified further, e. g. by way of ionic exchange chromatography. Thus, it is possible to obtain a linear ssNA fragment which is ready to be packaged into virus like particles or capsids. The invention will be described further by way of examples. It is clear that the examples do not restrict the subject matter of the present invention.

### Example:

A plasmid was provided as shown in figure 1 containing: From 5' to 3': a Pstl restriction site followed by a Pvull restriction site, followed directly by a functional AAV2 ITR sequence as present on common AAV vector plasmids, further followed by a cytomegalovirus promoter, followed by the coding sequence for fluorescence reporter protein mKate2, followed by the polyadenylation signal of the human growth hormone as shown in SEQ ID No. 1. The sequence is followed further by an inverse complement of the 5' ITR sequence harboring a deletion of ITR nucleobases 5'-TTT-GCCCGGGC (SEQ ID No. 2), and by the Pvull and Pstl restriction sites. Further 3' sequence elements are a pBR322 origin of replication, an ampicillin resistance gene and a f1 origin of replication.

In a first step, enzymatic digestion of the plasmid was conducted using Pstl as follows: 10 µg of the before described plasmid were digested with 2 µl of commercially available Pstl-HF (New England Biolabs) per the manufacturer's instructions in a final volume of 50 µl and in the buffer suggested by the manufacturer for at least 2 hours at 37 °C.

The fragments obtained were separated by electrophoresis, thus isolating the double stranded nucleic acid fragment containing the nucleic acid fragment with viral ITR sequences and the GOI. Separation was performed in an agarose gel in Tris-Acetate-EDTA buffer. Gel extraction was performed with a commercially available kit (NucleoSpin Gel and PCR Clean-up, Macherey-Nagel). The isolated fragment was then recirculated using commercially available T4 ligase (Thermo Scientific). A 200 µl reaction mixture was prepared containing 300 ng of isolated fragment, 10 µl of T4 ligase and commercially available phi29 polymerase reaction buffer (New England Biolabs) supplemented to a final concentration of 1 mM ATP and 10 mM DTT. The mixture was incubated for 1 hour at room temperature.

The circular double stranded nucleic acid fragments thus obtained joint at the compatible ends formed by digestion with Pstl were then hybridized with a primer 5'-GGCGGAGTTGTTACGACA (SEQ ID. No. 3) by adding the primer to a final concentration of 180 nM and incubating the reaction mixture at 95 °C for two minutes and cooling it back to room temperature. A polymerase with high processability and strong strand displacement activity, here phi29 polymerase, was added to the circular DNA fragments to start the amplification. For amplification, the mixture was brought to a final volume of 300 µl including 0.7 mM dNTPs, 0.3 mg/ml BSA and 5 µl of commercial phi29 polymerase (New England Biolabs). A second amplification was performed in presence of 25 ng/µl T4gp32 SSB (NEB). By rolling circle amplification at 30 °C for two days, single stranded multimers of the DNA fragments were obtained whereby the flanking ITR's are present as single strands covalently bound with each other in short distance. Next, by heating and slow cooling, DNA hairpins formed with a recognition site for the second restriction enzyme, here Pvull. The multimeric product obtained after the rolling circle amplification was digested with Pvull by bringing the reaction mixture to a final volume of 600 µl, including 15 µl of Pvull-HF restriction enzyme (New England Biolabs) and the buffer recommended by the manufacturer. The mixture was incubated at 37 °C for 300 minutes to obtain a linear ssNA fragment containing the two ITR sequences flanking the GOI. These fragments were purified by a silica-based PCR cleanup kit (Macherey Nagel). The multimeric product obtained from the second amplification was additionally digested with Ndel (NEB).

The fragments obtained by the method from the example were subsequently analyzed by denaturing polyacrylamide gel electrophoreses. Samples were denatured by heating in 8 M urea solution and separated on an 8 % acrylamide gel containing 8 M urea in Tris-borate EDTA buffer run at 6 V/cm for six hours. RotiSafe gel stain (Carl Roth) was used to visualize the fragments on a blue light table. The digested second amplification product was analyzed on an agarose gel in Tris-acetate-EDTA buffer run at 120 V for 50 minutes and visualized with SYBR Gold (Thermo).

The biological activity of the linear ssNA fragment containing the two ITR sequences flanking the fluorescence reporter gene in terms of transfection and virus production was tested in cell culture experiments. Adherent HEK293 cells were grown in commercial DMEM culture media containing 10 % fetal calf serum. Cells were seeded at 52,000 cells per cm² culture surface one day before transfection experiments. The next day, cells were transfected by the calcium phosphate transfection method. For transfection of 3 x 10⁶ seeded cells, a total of 15 µg DNA was diluted in 0.25 ml of 0.3 M CaCl₂ solution. The DNA solution was added dropwise to an equal volume of HEPES-buffered saline containing disodium phosphate (50 mM HEPES, 1.5 mM disodium phosphate, 280 mM sodium chloride, pH 7.05). The resulting mixture was slowly and dropwise added to the cells. Either only the DNA fragment obtained by the method described in the example was used for transfection, or a molar 1:1:1 ratio of the fragment from the example, a plasmid encoding the adeno-associated virus capsid proteins and its helper-genes (pRepCap, e.g. according to SEQ ID No. 5) and an adeno-virus helper plasmid (pHelper, Agilent technologies AAV helper free system) to produce virus particles. pRepCap and pHelper are well-known to the prior art.

Further analysis of the transfected cells was performed after three days of subsequent cultivation, either by fluorescence imaging using a fluorescence microscope with a filter set suitable for the fluorescence reporter mKate2, or by quantitative PCR to determine the titer of produced virus particles. Samples for quantitative PCR were prepared by lysis of transfected cells. For this, cells were harvested by scraping and pelleted by centrifugation. The pellet was resuspended in Tris-buffered saline and subjected to three freeze-thaw cycles. Cellular debris was pelleted by centrifugation and the supernatant containing the virus particles was collected and filtered sterile. A 2.5 µl sample of this lysate was incubated with an equal volume of DNasel (New England Biolabs) in a 25 µl reaction volume as described by the manufacturer, to digest all DNA not packaged in virus capsids. The mixture was then diluted 5-fold with distilled water and 5 µl of the dilution was used in a 20 µl qPCR reaction with primers 5'- GGGACTTTCCTACTTGGCA (SEQ ID. No. 4) and 5'- GGCGGAGTTGTTAC-GACA (SEQ ID. No. 3).

### Results:

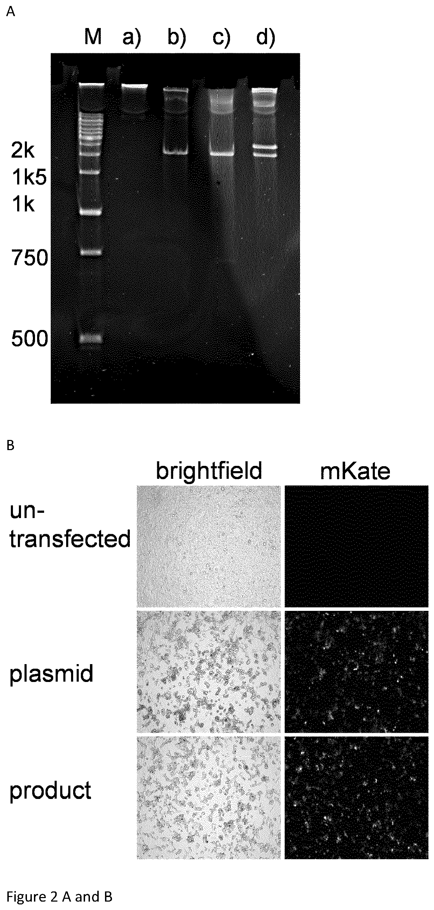

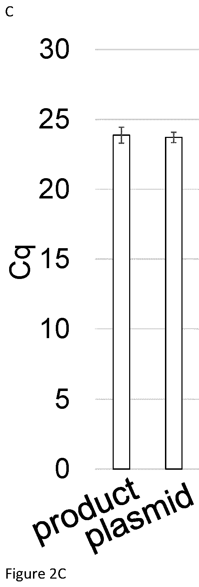

The results from the example are described referring to figure 2.

Scheme (A) of figure 2 shows a denaturing PAGE of the resulting DNA fragments and intermediate steps of the example process to generate linear, ssNA fragments containing two viral inverted terminal repeat sequences flanking a gene of interest according to the present invention. Lane d) of the gel shows the Pstl digestion of the plasmid according to step b) of the current invention. Two bands are clearly visible with the smaller of which being the ITR containing fragment with a size of 2.2 kb and the larger fragment being the plasmid backbone. Lane a) shows the result of the rolling circle amplification present as high molecular weight fragments unable to migrate into the gel. Lane b) shows the sample of lane a), but digested with Pvull, leading to the release of the desired single stranded fragment. Lane c) shows the final product purified by a silica-based purification method.

Fragments from the Pvull-/Ndel-digestion run on an agarose gel were analyzed for bands attributable to double digestion as a hint on double-stranded products from the RCA. This is, because Pvull should always release the fragments according to the present invention, but Ndel which cuts inside the GOI should only cut when its recognition sequence is present as a double-strand. Indeed, no signs of double-digestion were seen on the gel, proving the single-strand purity of the product.

The single stranded linear nucleic acid fragment according to the present invention obtained above had biological activity in HEK293 cells. Scheme (B) of figure 2 shows the result of the transfection experiment where HEK293 were either not transfected as a control (upper two panels), transfected with the plasmid according to the present invention (middle two panels), or transfected with an equal molar amount of the linear, single stranded fragments according to the present invention (lower two panels). The production of the reporter protein mKate2 was determined in all three cases by fluorescence imaging (panels on the right). As evident from the presented images, fluorescence emission as a measure of recombinant protein production is comparable between the two transfections, demonstrating that the biological activity of the linear single stranded fragment in terms of transfectability and protein expression equals that of its progenitor plasmid, while at the same time avoiding the transfection of prokaryotic DNA.

To identify further whether the linear ssNA fragments containing the two viral ITR sequences flanking the gene of interest are suitable for production of virus particles, a test was conducted based on a three plasmid system as described by Matsushita T. et al, and by Xiao X. et al, see above. The progenitor plasmid according to the present invention was used as reference. After a three-day production in HEK293 cells, the amount of DNasel resistant particles was determined as a measure of qPCR quantification cycle (Cq). As shown in scheme (C) of figure 2, comparable amounts are present in the control as well as in the virus sample produced with the ssNA fragment according to the present invention. That is, the biological functions of the nucleic acid according to the present invention, namely, competence in virus particle production and gene expression, as well as transfectability are similar to plasmids currently used to produce recombinant AAV, while at the same time avoiding the transfection of prokaryotic DNA.

## Claims

1. Method for the *in vitro* generation of genetically engineered, linear, single-stranded nucleic acid (ssNA) fragments containing two adeno-associated viral inverted terminal repeat (AAV ITR) sequences flanking a gene of interest (GOI), being essentially free of bacterial nucleic acid sequences, comprising the steps of:
a) Providing a bacterial nucleic acid plasmid containing a nucleic acid fragment containing the two AAV ITR sequences and the GOI;
b) Enzymatic digestion of the plasmid with a first restriction enzyme (1) or a combination of at least two restriction enzymes (1) and (4) generating the double stranded nucleic acid fragment with terminal compatible ends;
c) isolation of the linear double stranded nucleic acid fragment containing the nucleic acid fragment with AAV ITR sequences and GOI;
d) Recirculation of the isolated linear double stranded nucleic acid fragment of step c) to obtain a double stranded circular nucleic acid fragment joint at the compatible ends formed in step b);
e) Amplification of the circulated double stranded fragment of d) containing two AAV ITRs and the GOI by a nucleic acid amplification technique generating single-stranded nucleic acids comprising at least two covalently linked copies, reverse complement copies, of the template strand;
f) Enzymatic digestion of the amplification product obtained in step e) with a second restriction enzyme (2) or two different restriction enzymes (2) and (3) to obtain linear ssNA sequences comprising said nucleic acid fragment of the two AAV ITR sequences and the GOI; and
g) Purification of said linear ssNA fragment obtained in step f) comprising the nucleic acid fragment of the two AAV ITR sequences and the GOI.

2. The method for the *in vitro* generation of genetically engineered, linear, single stranded viral nucleic acid fragments according to claim 1, wherein the first restriction enzyme (1) and fourth restriction enzyme (4) is a sticky end generating enzyme, like Pstl.

3. The method for the *in vitro* generation of genetically engineered, linear, single-stranded viral nucleic acid fragments according to claim 1 or 2, wherein the second restriction enzyme (2) and third restriction enzyme (3) is a blunt end cutting restriction enzyme, like Pvull.

4. The method for the *in vitro* generation of genetically engineered, linear, single-stranded viral nucleic acid fragments according to any one of claims 1 to 3, wherein the plasmid containing viral nucleic acid fragments contains the sequence elements:
a) A recognition sequence for a restriction enzyme (1) generating ends, like sticky ends, being compatible to the generated ends of g);
b) Downstream a recognition sequence for a restriction enzyme (2) generating ends, like sticky ends, being compatible to the generated ends of f), and said recognition sequence being different to the recognition sequence of a) and g);
c) Downstream a functional AAV ITR sequence;
d) Downstream the GOI;
e) Downstream a functional AAV ITR sequence being identical or different to the functional ITR sequence of c);
f) Downstream a recognition sequence for a restriction enzyme (3) generating ends being compatible generated ends of b) but different to the ends of a) and g), whereby the restriction enzyme (3) may be identical or different to the restriction enzyme (2) of b);
g) Downstream a recognition sequence for a restriction enzyme (4) generating ends, being compatible to the ends generated in a), whereby said restriction enzyme (4) may be identical or different to the restriction enzyme (1); whereby if restriction enzymes are used, that can cut at different distances relative to the recognition site, the recognition sequences in a) and b) as well as in f) and g) can be at the same position and up to all recognition sequences can be identical.

5. The method for the *in vitro* generation of genetically engineered, linear, single-stranded viral nucleic acid fragments according to any one of the preceding claims, wherein i) isolation of the double-stranded nucleic acid fragment obtained in step d) containing the viral nucleic acid fragment and/or ii) purification of the linear ssNA fragment comprising viral nucleic acid fragments in step g), is conducted by electrophoreses or ion exchange chromatography or silica adsorption chromatography.

6. The method for the *in vitro* generation of genetically engineered, linear, single-stranded viral nucleic acid fragments according to any one of the preceding claims, wherein recirculation of the nucleic acid fragment in step d) is conducted by a ligase, in particular, a T4ligase.

7. The method for the *in vitro* generation of genetically engineered, linear, single-stranded viral nucleic acid fragments according to any one of the preceding claims, wherein the amplification step e) is performed enzymatically by a polymerase; preferably, the polymerase is the phi29 polymerase; and/or preferably the amplification is an isothermal reaction.

8. The method for the *in vitro* generation of genetically engineered, linear, single-stranded viral nucleic acid fragments according to any one of the preceding claims, wherein hybridization of an oligonucleotide for amplification in step e) is conducted by heat denaturation of said circular double stranded nucleic acid fragment and subsequent cooling of the reaction mixture.

9. The method for the *in vitro* generation of genetically engineered, linear, single-stranded viral nucleic acid fragments according to any one of the preceding claims, wherein in step e) the amplification product obtained by amplification of the single stranded circular nucleic acid fragment, in particular, the DNA fragment, is heat denatured and cooled to allow hybridization of adjacent inverted terminal repeat and restriction site sequences to form a double stranded hairpin containing a functional restriction site.

10. A plasmid comprising the following nucleic acid sequence elements:
a) A recognition sequence for a first restriction enzyme (1) generating ends, like sticky ends, being compatible to the generated ends of g);
b) Downstream a recognition sequence for a second restriction enzyme (2) and said recognition sequence being different to the recognition sequence of a) and g), whereby the recognition sequence is selected from the sequences recognized by Type IIS enzymes which would generate a 3'-OH towards the functional ITR directly upstream or inside of the ITR sequence of c);
c) Downstream a functional AAV ITR sequence;
d) Downstream the GOI;
e) Downstream a functional AAV ITR sequence being identical or different to the functional ITR sequence of c);
f) Downstream a recognition sequence for a third restriction enzyme (3) different to the recognition sequences of a) and g); and the recognition sequence is the reverse complement of the sequence of b);
g) Downstream a recognition sequence for a fourth restriction enzyme (4) generating ends, being compatible to the ends generated in a), whereby said fourth restriction enzyme (4) may be identical or different to the first enzyme (1).

11. The plasmid according to claim 10, wherein the recognition sequence of the restriction enzyme (2) and (3) is selected from the sequence set of Type IIS enzymes selected from Aarl, Acc361, AcIWI, Acul, Alw261, AIwI, AsuHPI, Bbsl, Bbvl, Bccl, BceAI, BciVI, BcoDI, BfuAI, Bful, Bmrl, Bmsl, Bmul, Bpil, Bpml, BpuEI, Bsal, Bse3DI, BseGI, BseMII, BseRI, BseXI, Bsgl, BsIFI, BsmBI, BsmFI, Bso31I, BspCNI, BspMI, BspPI, BspQI, BspTNI, BsrDI, Bst61, BstF51, BstMAI, BstV1I, BstV2I, Bsul, BtgZI, BtsCI, BtsiIMutI, Btsal, Bvel, Csel, Eam1104I, Earl, Ecil, Eco57I, Esp3I, Faql, Faul, Fokl, Gsul, Hgal, Hphl, HpyAV, Lgul, Lsp1109I, LweI, Mboll, Mfel, Mlyl, Mmel, NmeAIII, PciSI, Plel, Ppsl, Sapl, Schl, SfaNI, Taqll, TspDTI or TspGWI.

## Patentansprüche

1. Ein Verfahren zur *in vitro* Herstellung von genetisch konstruierten, linearen, einzelsträngigen Nukleinsäure (ssNA) Fragmenten, die zwei Adeno-assoziierte Virus Inverted Terminal Repeat (AAV ITR) Sequenzen enthalten, die ein Gen von Interesse (GOI) flankieren, und die im Wesentlichen frei von bakteriellen Nukleinsäuresequenzen sind, umfassend die Schritte:
a) Bereitstellen eines bakteriellen Nukleinsäureplasmids, enthaltend ein Nukleinsäurefragment, enthaltend die zwei AAV ITR Sequenzen und das GOI;
b) enzymatischer Verdau des Plasmids mit einem ersten Restriktionsenzym (1) oder einer Kombination von mindestens zwei Restriktionsenzymen (1) und (4), die das doppelsträngige Nukleinsäurefragment mit terminalen kompatiblen Enden herstellen;
c) Isolieren des linearen doppelsträngigen Nukleinsäurefragments, enthaltend das Nukleinsäurefragment mit AAV ITR Sequenzen und GOI;
d) Rezirkulation des isolierten linearen doppelsträngigen Nukleinsäurefragments von Schritt c), um ein doppelsträngiges zirkuläres Nukleinsäurefragment zu erhalten, das an den kompatiblen Enden, die in Schritt b) gebildet wurden, verbunden ist;
e) Amplifikation des zirkulären doppelsträngigen Fragments von d), enthaltend die zwei AAV ITRs und das GOI durch Nukleinsäureamplifikationstechnik, die einzelsträngige Nukleinsäuren herstellt, die mindestens zwei kovalent verbundene Kopien, reverse komplemente Kopien des Template Stranges umfassen;
f) enzymatischer Verdau des Amplifikationsprodukts , das in Schritt e) erhalten wurde, mit einem zweiten Restriktionsenzym (2) oder zwei verschiedenen Restriktionsenzymen (2 ) und (3), um lineare ssNA Sequenzen zu erhalten, die das Nukleinsäurefragment der zwei AAV ITR Sequenzen und des GOI umfassen; und
g) Purifikation des linearen ssNA Fragments, das in Schritt f) erhalten wurde, umfassend das Nukleinsäurefragment der zwei AAV ITR Sequenzen und des GOI.

2. Das Verfahren für die *in vitro* Herstellung von genetisch konstruierten linearen einzelsträngigen viralen Nukleinsäurefragmenten nach Anspruch 1, wobei das erste Restriktionsenzym (1) und das vierte Restriktionsenzym (4) ein sticky end Enzym, wie Pstl ist.

3. Das Verfahren für die *in vitro* Herstellung von genetisch konstruierten linearen einzelsträngigen viralen Nukleinsäurefragmenten nach Anspruch 1 oder 2, wobei das zweite Restriktionsenzym (2) und das dritte Restriktionsenzym (3) ein blunt end Schneide-Restriktionsenzym, wie Pvull ist.

4. Das Verfahren für die *in vitro* Herstellung von genetisch konstruierten linearen einzelsträngigen viralen Nukleinsäurefragmenten nach einem der Ansprüche 1 bis 3, wobei das Plasmid, das die viralen Nukleinsäurefragmente enthält, die folgenden Sequenzelemente enthält:
a) eine Erkennungssequenz für ein Restriktionsenzym (1), das Enden, wie sticky ends, erzeugt, die kompatibel zu den in g) erzeugten Enden sind;
b) downstream eine Erkennungssequenz für ein Restriktionsenzym (2), das Enden, wie sticky ends, erzeugt, die kompatibel zu den in f) erzeugten Enden sind, und wobei jene Erkennungssequenz verschieden ist zu der Erkennungssequenz von a) und g);
c) downstream eine funktionale AAV ITR Sequenz;
d) downstream das GOI;
e) downstream eine funktionale AAV ITR Sequenz, die identisch oder verschieden zu der funktionalen ITR Sequenz von c) ist;
f) downstream eine Erkennungssequenz für ein Restriktionsenzym (3), das Enden erzeugt, die kompatibel zu den in b) erzeugten Enden sind, aber verschieden zu den Enden von a) und g) sind, wobei das Restriktionsenzym (3) identisch oder verschieden zu dem Restriktionsenzym (2) von b) sein kann;
g) downstream eine Erkennungssequenz für ein Restriktionsenzym (4), das Enden erzeugt, die kompatibel zu den in a) erzeugten Enden sind, wobei das Restriktionsenzym (4) identisch oder verschieden zu dem Restriktionsenzym (1) sein kann; wobei wenn Restriktionsenzyme verwendet werden, die in verschiedenen Distanzen relativ zur Erkennungsstelle schneiden können, die Erkennungssequenzen in a) und b), sowie in f) und g) an derselben Position sein können und alle Erkennungssequenzen identisch sein können.

5. Das Verfahren für die *in vitro* Herstellung von genetisch konstruierten linearen einzelsträngigen viralen Nukleinsäurefragmenten nach einem der vorhergehenden Ansprüche, wobei (i) die Isolation des doppelsträngigen Nukleinsäurefragments, das in Schritt d) erhalten wurde, das das virale Nukleinsäurefragment enthält und/ oder (ii) Purifikation des linearen ssNA Fragments, umfassend die viralen Nukleinsäurefragmente in Schritt g) durch Elektrophorese oder Ionenaustauschchromatographie oder Silikaadsorptionschromatographie durchgeführt wird.

6. Das Verfahren für die *in vitro* Herstellung von genetisch konstruierten linearen einzelsträngigen viralen Nukleinsäurefragmenten nach einem der vorhergehenden Ansprüche, wobei die Rezirkulation des Nukleinsäurefragments in Schritt d) durch Ligase, insbesondere T4 Ligase durchgeführt wird.

7. Das Verfahren für die *in vitro* Herstellung von genetisch konstruierten linearen einzelsträngigen viralen Nukleinsäurefragmenten nach einem der vorhergehenden Ansprüche, wobei die Amplifikation in Schritt e) enzymatisch durch Polymerase durchgeführt wird; wobei bevorzugt die Polymerase die phi29 Polymerase ist und/ oder wobei bevorzugt die Amplifikation eine isothermale Reaktion ist.

8. Das Verfahren für die *in vitro* Herstellung von genetisch konstruierten linearen einzelsträngigen viralen Nukleinsäurefragmenten nach einem der vorhergehenden Ansprüche, wobei die Hybridisierung eines Oligonukleotids für die Amplifikation in Schritt e) durch Hitzedenaturierung des zirkulären doppelsträngigen Nukleinsäurefragments und anschließende Kühlung der Reaktionsmischung durchgeführt wird.

9. Das Verfahren für die *in vitro* Herstellung von genetisch konstruierten linearen einzelsträngigen viralen Nukleinsäurefragmenten nach einem der vorhergehenden Ansprüche, wobei in Schritt e) das Amplifikationsprodukt, das durch Amplifikation des einzelsträngigen zirkulären Nukleinsäurefragments erhalten wurde, insbesondere das DNA Fragment, hitzedenaturiert wird und gekühlt wird, um die Hybridisierung von anliegenden Inverted Terminal Repeats und Restriktionsstellensequenzen zu ermöglichen, um doppelsträngige Haarnadeln zu bilden, die eine funktionale Restriktionsstelle enthalten.

10. Ein Plasmid umfassend die folgenden Nukleinsäureelemente:
a) eine Erkennungssequenz für ein erstes Restriktionsenzym (1), das Enden, wie sticky ends, erzeugt, die kompatibel zu den in g) erzeugten Enden sind;
b) downstream eine Erkennungssequenz für ein zweites Restriktionsenzym (2) und wobei die Erkennungssequenz verschieden zu der Erkennungssequenz von a) und g) ist, wobei die Erkennungssequenz ausgewählt ist aus den Sequenzen, die von Type IIS Enzymen erkannt werden, die ein 3'OH in Richtung des funktionalen ITRs direkt upstream oder innerhalb der ITR Sequenz erzeugen würden;
c) downstream eine funktionale AAV ITR Sequenz;
d) downstream das GOI;
e) downstream eine funktionale AAV ITR Sequenz, die identisch oder verschieden zu der funktionalen ITR Sequenz von c) ist;
f) downstream eine Erkennungssequenz für ein drittes Restriktionsenzym (3), die verschieden zu den Erkennungssequenzen von a) und g) ist; und wobei die Erkennungssequenz das reverse Komplement zu der Sequenz von b) ist;
g) downstream eine Erkennungssequenz für ein viertes Restriktionsenzym (4), das Enden erzeugt, die kompatibel zu den in a) erzeugten Enden sind, wobei das vierte Restriktionsenzym (4) identisch oder verschieden zu dem ersten Enzym (1) sein kann.

11. Das Plasmid nach Anspruch 10, wobei die Erkennungssequenz der Restriktionsenzyme (2) und (3) ausgewählt ist aus dem Sequenzset von Type IIS Enzymen ausgewählt aus Aarl, Acc361, AcIWI, Acul, Alw261, Alwl, AsuHPl, Bbsl, Bbvl, Bccl, BceAI, BciVI, BcoDI, BfuAI, Bful, Bmrl, Bmsl, Bmul, Bpil, Bpml, BpuEl, Bsal, Bse3DI, BseGI, BseMII, BseRI, BseXI, Bsgl, BsIFI, BsmBI, BsmFl, Bso31I, BspCNI, BspMI, BspPI, BspQl, BspTNI, BsrDI, Bst61, BstF51, BstMAI, BstV1I, BstV2I, Bsul, BtgZI, BtsCl, BtslMutl, Btsal, Bvel, Csel, Eam1104I, Earl, Ecil, Eco57I, Esp3I, Faql, Faul, Fokl, Gsul, Hgal, Hphl, HpyAV, Lgul, Lsp1109I, Lwel, Mboll, Mfel, Mlyl, Mmel, NmeAlll, PciSI, Plel, Ppsl, Sapl, Schl, SfaNI, Taqll, TspDTI or TspGWI.

## Revendications

1. Procédé de génération in vitro de fragments d'acide nucléique simple brin (ssNA) linéaires génétiquement modifiés contenant deux séquences de répétition terminale inversée virale adéno-associée (AAVITR) flanquant un gène d'intérêt (GOI), étant essentiellement exempts de séquences d'acide nucléique bactérien, comprenant les étapes de :
a) la fourniture d'un plasmide d'acide nucléique bactérien contenant un fragment d'acide nucléique contenant les deux séquences ITR AAV et le GOI ;
b) la digestion enzymatique du plasmide avec une première enzyme de restriction (1) ou une combinaison d'au moins deux enzymes de restriction (1) et (4) générant le fragment d'acide nucléique double brin avec des extrémités compatibles terminales ;
c) l'isolement du fragment d'acide nucléique double brin linéaire contenant le fragment d'acide nucléique avec des séquences AAV ITR et le GOI ;
d) la recirculation du fragment d'acide nucléique double brin linéaire isolé de l'étape c) pour obtenir un joint de fragment d'acide nucléique double brin circulaire aux extrémités compatibles formées à l'étape b) ;
e) l'amplification du fragment double brin circulaire de d) contenant deux AAV ITR et le GOI par une technique d'amplification d'acide nucléique générant des acides nucléiques simple brin comprenant au moins deux copies liées par covalence, copies de complément inverse, du brin modèle ;
f) la digestion enzymatique du produit d'amplification obtenu à l'étape e) avec une deuxième enzyme de restriction (2) ou deux enzymes de restriction (2) et (3) différentes pour obtenir des séquences ssNA linéaires comprenant ledit fragment d'acide nucléique des deux séquences AAV ITR et le GOI ; et
g) la purification dudit fragment ssNA linéaire obtenu à l'étape f) comprenant le fragment d'acide nucléique des deux séquences AAV ITR et le GOI.

2. Procédé de génération in vitro de fragments d'acide nucléique viral simple brin linéaire génétiquement modifiés selon la revendication 1, dans lequel la première enzyme de restriction (1) et la quatrième enzyme de restriction (4) sont une enzyme générant une extrémité collante, comme Pstl.

3. Procédé de génération in vitro de fragments d'acide nucléique viral simple brin linéaire génétiquement modifiés selon la revendication 1 ou 2, dans lequel la deuxième enzyme de restriction (2) et la troisième enzyme de restriction (3) sont une enzyme de restriction de coupe à extrémité émoussée, comme Pvull.

4. Procédé de génération in vitro de fragments d'acide nucléique viral simple brin linéaire génétiquement modifiés selon l'une quelconque des revendications 1 à 3, dans lequel le plasmide contenant des fragments d'acide nucléique viral contient les éléments de séquence suivants :
a) une séquence de reconnaissance pour une enzyme de restriction (1) générant des extrémités, telles que des extrémités collantes, étant compatibles avec les extrémités générées de g) ;
b) en aval, une séquence de reconnaissance pour une enzyme de restriction (2) générant des extrémités, telles que des extrémités collantes, étant compatibles avec les extrémités générées de f), et ladite séquence de reconnaissance étant différente de la séquence de reconnaissance de a) et g) ;
c) en aval, une séquence AAV ITR fonctionnelle ;
d) en aval, le GOI ;
e) en aval, une séquence AAV ITR fonctionnelle identique ou différente de la séquence ITR fonctionnelle de c) ;
f) en aval, une séquence de reconnaissance pour une enzyme de restriction (3) générant des extrémités compatibles avec les extrémités générées de b) mais différentes des extrémités de a) et g), l'enzyme de restriction (3) pouvant être identique ou différente de l'enzyme de restriction (2) de b) ;
g) en aval, une séquence de reconnaissance pour une enzyme de restriction (4) générant des extrémités, étant compatible avec les extrémités générées en a), ladite enzyme de restriction (4) pouvant être identique ou différente de l'enzyme de restriction (1) ; s'il est utilisé des enzymes de restriction pouvant couper à différentes distances par rapport au site de reconnaissance, les séquences de reconnaissance de a) et b) ainsi que de f) et g) pouvant être à la même position et jusqu'à toutes les séquences de reconnaissance pouvant être identiques.

5. Procédé de génération in vitro de fragments d'acide nucléique viral simple brin linéaire génétiquement modifiés selon l'une quelconque des revendications précédentes, dans lequel i) l'isolement du fragment d'acide nucléique double brin obtenu à l'étape d) contenant le fragment d'acide nucléique viral et/ou ii) la purification du fragment ssNA linéaire comprenant des fragments d'acide nucléique viral à l'étape g), sont réalisés par électrophorèse ou chromatographie par échange d'ions ou chromatographie par adsorption de silice.

6. Procédé de génération *in vitro* de fragments d'acide nucléique viral simple brin linéaire génétiquement modifiés selon l'une quelconque des revendications précédentes, dans lequel la recirculation du fragment d'acide nucléique à l'étape d) est réalisée par une ligase, en particulier une T4ligase.

7. Procédé de génération in vitro de fragments d'acide nucléique viral simple brin linéaire génétiquement modifiés selon l'une quelconque des revendications précédentes, dans lequel l'étape d'amplification e) est réalisée enzymatiquement par une polymérase ; de préférence, la polymérase est la polymérase phi29 ; et/ou de préférence l'amplification est une réaction isotherme.

8. Procédé de génération in vitro de fragments d'acide nucléique viral simple brin linéaire génétiquement modifiés selon l'une quelconque des revendications précédentes, dans lequel l'hybridation d'un oligonucléotide pour amplification à l'étape e) est réalisée par dénaturation thermique dudit fragment d'acide nucléique double brin circulaire et refroidissement ultérieur du mélange réactionnel.

9. Procédé de génération in vitro de fragments d'acide nucléique viral simple brin linéaire génétiquement modifiés selon l'une quelconque des revendications précédentes, dans lequel, à l'étape e), le produit d'amplification obtenu par amplification du fragment d'acide nucléique circulaire simple brin, en particulier du fragment d'ADN, est dénaturé à chaud et refroidi pour permettre l'hybridation de séquences de sites de répétition et de restriction terminales inverses adjacentes pour former une épingle à cheveux double brin contenant un site de restriction fonctionnel.

10. Plasmide comprenant les éléments de séquence d'acide nucléique suivants :
a) une séquence de reconnaissance pour une première enzyme de restriction (1) générant des extrémités, telles que des extrémités collantes, étant compatible avec les extrémités générées de g) ;
b) en aval, une séquence de reconnaissance pour une deuxième enzyme de restriction (2) et ladite séquence de reconnaissance étant différente de la séquence de reconnaissance de a) et g), la séquence de reconnaissance étant choisie parmi les séquences reconnues par les enzymes de type IIS qui généreraient un 3'-OH vers l'ITR fonctionnel directement en amont ou à l'intérieur de la séquence ITR de c) ;
c) en aval, une séquence AAV ITR fonctionnelle ;
d) en aval, le GOI ;
e) en aval, une séquence AAV ITR fonctionnelle identique ou différente de la séquence ITR fonctionnelle de c) ;
f) en aval, une séquence de reconnaissance pour une troisième enzyme de restriction (3) différente des séquences de reconnaissance de a) et g) ; et la séquence de reconnaissance est le complément inverse de la séquence de b) ;
g) en aval, une séquence de reconnaissance pour une quatrième enzyme de restriction (4) générant des extrémités, étant compatibles avec les extrémités générées de a), ladite quatrième enzyme de restriction (4) pouvant être identique ou différente de la première enzyme (1).

11. Plasmide selon la revendication 10, dans lequel la séquence de reconnaissance des enzymes de restriction (2) et (3) est choisie parmi l'ensemble de séquences d'enzymes de type IIS choisies parmi Aarl, Acc361, AclWI, Aiguille, Alw261, Alwl, AsuHPI, Bbsl, Bbvl, Bccl, BceAl, BciVI, BcoDI, BfuAI, Bful, Bmrl, Bmsl, Bmul, Bpil, Bpml, BpuEl, Bsal, Bse3DI, BseGI, BseMII, BseRl, BseXI, Bsgl, BsIFI, BsmBl, BsmFl, Bso31I, BspCNI, BspMI, BspPl, BspQl, BspTNI, BsrDI, Bst61, BstF51, BstMAI, BstV1I, BstV2I, Bsul, BtgZl, BtsCl, BtslMutl, Btsal, Bvel, Cel, Eam1104I, Earl, Ecil, Eco57I, Esp3I, Faql, Faul, Fokl, Gsul, Hgal, Hphl, HpyAV, Lgul, Lsp1109I, Lwel, Mboll, Mfel, Mlyl, Mmel, NmeAIII, PciSI, Plel, Ppsl, Sapl, Schl, SfaNI, Taqll, TspDTI et TspGWI.
